Europäisches Patentamt

European Patent Office

Office européen des brevets

(19)

(11) **EP 1 153 621 A1**

(12) **EUROPÄISCHE PATENTANMELDUNG**

(43) Veröffentlichungstag:
  **14.11.2001 Patentblatt 2001/46**

(51) Int Cl.[7]: **A61L 27/12**, A61L 24/02,
  A61K 6/033

(21) Anmeldenummer: **00110045.2**

(22) Anmeldetag: **12.05.2000**

(84) Benannte Vertragsstaaten:
  **AT BE CH CY DE DK ES FI FR GB GR IE IT LI LU
  MC NL PT SE**
  Benannte Erstreckungsstaaten:
  **AL LT LV MK RO SI**

(71) Anmelder: **MERCK PATENT GmbH
  64293 Darmstadt (DE)**

(72) Erfinder:
  • **Wenz, Robert, Dr.
    61206 Wöllstadt (DE)**
  • **Driessens, Ferdinand, Prof.
    Ohe en Laak (NL)**

(54) **Biozemente auf TCP-PHA-Basismischung mit verbesserter Kompressionsfestigkeit**

(57)   Die Erfindung beschreibt biologisch abbaubare Calciumphosphat-Zemente, insbesondere Mischungen aus calciumphosphathaltigen Pulvern unterschiedlicher stöchiometrischer Zusammensetzung, wobei der enthaltende präzipitierte Hydroxylapatit ein Kation-defizienter Hydroxylapatit der Formel 1 ist, wodurch die Mischungen verbesserte Eigenschaften hinsichtlich Kompressionsfestigkeit zeigen.

EP 1 153 621 A1

**Beschreibung**

Gegenstand der älteren deutschen Patentanmeldung P 198 13 614.5 sind biologisch abbaubare Calciumphosphat-Zemente, insbesondere Mischungen aus calciumphosphathaltigen Pulvern unterschiedlicher stöchiometrischer Zusammensetzung mit verbesserten Eigenschaften. Diese Mischungen enthalten alle Tricalciumphosphat (TCP) und eine oder mehrere andere phosphathaltige anorganische Verbindungen in unterschiedlicher Zusammensetzung, wobei der TCP-Anteil in einem wohl definierten Partikelgrössenbereich vorliegt. Erfindungswesentlich war dabei, dass ein bestimmter Anteil von Feinpartikeln (ca. 1 - 40 μm) und Feinstpartikeln (0.1 - 1μm) neben einen bestimmten Anteil von Grobpartikeln (40 - 300 μm) vorliegen muss.

Die Lehre der vorliegenden Erfindung geht von der Aufgabe aus, biologisch abbaubare Calciumphosphat-Zemente der geschilderten Art weiterhin zu verbessern. Hierbei stehen insbesondere die folgenden Erwägungen im Vordergrund:

Bis Ende 1997 waren nur Prototypen des Biozement D (Zusammensetzung siehe DE 19813614.5) bekannt. Charakteristisch für diese Prototypen waren ihre ausreichenden Eigenschaften in Bezug auf Mischzeit, Kohäsionszeit, Doughtime, initiale und finale Aushärtezeit nach ASTM C266, aber die Kompressionsfestigkeit erreichte niemals Werte größer 50 MPa. Sie lag damit lediglich im Bereich des trabekulären humanen Knochens (Driessens et al., Bioceramics 10 (1997) 279-282). Als Kristallisationskeim wurde handelsüblicher TCP verwendet (siehe DE 19813614.5)

Aufgabe der vorliegenden Erfindung war es daher, für die Biozemente Kompressionsfestigkeiten > 50 MPa zu erreichen, die bzgl. Festigkeit sogar im Bereich des kortikalen Knochens liegen, ohne dass diese Biozemente negative Veränderungen in Bezug auf Verarbeitungszeiten und Kohäsion zeigen.

Diese Aufgabe wird nun erfindungsgemäß durch Verwendung eines speziell hergestellten, präzipitierten Hydroxylapatit (PHA) gelöst, wobei dieser als Kristallisationskeim bzw. Nukleationsmittel zur Ausbildung des bei der Aushärtungsreaktion entstehenden karbonisierten Apatits aus Biozement D dient. Überraschenderweise können somit nach der Aushärtung Kompressionsfestigkeiten von 70-80 MPa erreicht werden (siehe Tab. 1 und Fig. 1)

Gegenstand der vorliegenden Erfindung ist somit eine Mischung von Pulvern geeignet zur Herstellung von resorbierbaren Calciumphosphat-Zementen, enthaltend Tricalciumphosphat (TCP), wobei 30-70 % der TCP-Teilchen eine Partikelgröße von 0.1 - 7 μm und 10 - 60 % eine Partikelgröße von 40 - 100 μm aufweisen, präzipitierter Hydroxylapaptit (PHA) und mindestens einer weiteren anderen phosphathaltigen anorganischen Verbindung, wobei der PHA ein Kation-defizienter Hydroxylapatit mit der Zusammensetzung

$$Ca_{8.75}V(Ca)_{1.25}[(HPO_4)_{5.5-x}(CO_3)_{0.5}](OH)_xV(OH)_{2-x}$$

mit Werten für x zwischen 0 und 2.

Es wurde herausgefunden, dass es sich bei dem während der Abbinde-und Aushärtungsphase bildenden Präzipitat aus Biozement D, um einen karbonisierten, Kation-defizienten Hydroxylapatit der o.g. Summenformel handelt. Dabei bedeuten V(Ca) und V(OH) Fehlstellen von Ca und OH im Kristallgitter. Die Werte für x hängen wiederum vom strukturell bedingten Wassergehalt des Apatits ab. Es stellte sich weiterhin heraus, dass die Struktur und Zusammensetzung des bisher als Nukleationskeim zur Herstellung des Biozements D-Prototyps, verwendeten PHA, das sogenannte TCP, erheblich von der des oben beschriebenen Kation-defizienten Hydroxylapatits abweicht. Das im Stand der Technik (siehe DE 198 13614) verwendete TCP enthält als Hauptphase Apatit, welcher aber nur sehr wenig Carbonat ($CO_2$-Gehalt < 0.2 %) und als Nebenphasen Monetit enthält. Hieraus folgte der Schluss einen stärker karbonisierten, präzipitierten Kation-defizienten Hydroxylapatit herzustellen, welcher eine ähnliche Struktur besitzt wie sie sich während der Abbindung und Aushärtung des Biozementes D einstellt. Ein solches Material sollte besser als Nukleationsmittel für die Reaktion des Biozementes D geeignet sein. Die Herstellung des PHA erfolgt einfachstenfalls durch Konversion dreier Salze nach folgender Reaktion:

$$8.25\ CaSO_4 \cdot 2H_2O + 5.5\ Na_3PO_4 \cdot 12H_2O + 0.5\ CaCO_3 \rightarrow$$

$$Ca_{8.75}V(Ca)_{1.25}[(HPO_4)_{5.5-x}(CO_3)_{0.5}](OH)_xV(OH)_{2-x} + 8.25\ Na_2SO_4$$

$$+ (82.5-x)\ H_2O$$

Anstelle des $CaSO_4 \cdot 2H_2O$ können auch andere Calciumsalze starker Säuren wie z.B. ein Anhydrat oder Hydrat von Calciumchlorid oder Calciumnitrat verwendet werden. Deren Nachteil liegt jedoch in einer hohen Stöchiometrieabweichung, so dass man nicht sicher voraussagen kann, wieviel Calcium proportional in den drei Salzen enthalten ist.

Um einen Kation-defizienten Hydroxylapatit zu erhalten, muss die Lösung einen pH zwischen 7 und 9, vorzugsweise zwischen 7 und 8 haben. Dies wird am besten durch Lösen von $Na_2HPO_4$ oder $K_2HPO_4$ oder deren Mischung in einer wässrigen Lösung erreicht, in die dann die drei o.g. Salze entsprechend gelöst werden.

Eine weitere Voraussetzung ist die Partikelgröße des PHA. Damit dieser als Nukleationsmittel im Biozement D, H oder auch F (siehe DE 19813614.5) geeignet ist, sollte die Partikelgröße zwischen 0.5 und 10 $\mu$m, vorzugsweise zwischen 0.5 und 5 $\mu$m liegen. Dies wird durch das Lösen von Magnesiumchlorid und/oder Magnesiumsulfat und/oder Magnesiumnitrat und/oder eines oder mehrere ihrer Hydrate in einer wässrigen Lösung erreicht, in welcher die Reaktion zum PHA ausgeführt wird und in welcher die Magesiumsalze, vorzugsweise vor dem Einmischen der drei Salze nach o.g. Gleichung gelöst werden. Nach Formung des Präzipitats von PHA in der Lösung, soll diese längere Zeit bei Raumtemperatur aufbewahrt werden um die Inkorporation der Carbonatanionen in das PHA zu vervollständigen. Jegliche Temperaturerhöhung muss vermieden werden um ein Kristallwachstum des Präzipitats zu vermeiden. Danach wird das Präzipitat aus der wässrigen Lösung z.B. durch Filtration oder Zentrifugation entfernt, wobei das Präzipitat mit einem Überschuss einer, einen neutralen Elektrolyten enthaltenden, wässrigen Lösung gewaschen wird, um die Natrium- und Sulfat-Ionen zu entfernen. Spuren dieser Ionen im PHA in der Größenordnung von < 0.1 Gew. % sind dabei akzeptabel. Als neutrale Elektrolyte werden vorzugsweise Na- oder K-Salze, in Form von Chloriden und/oder Sulfaten und/oder Nitraten und/oder eines oder mehrere ihrer Hydrate verwendet. Der Grund für die Verwendung dieser neutralen Elektrolyte in der Waschlösung liegt in der Verhinderung des Quellens und der Disproportionierung des Präzipitats. Nach der Spülung des Präzipitats wird dieses über Nacht bei 120° C getrocknet. Um eine Aggregation zu vermeiden, sollte die Trocknung nicht über 16 h liegen. Danach ist der so hergestellte PHA gebrauchsfertig zur Herstellung des endgültigen Biozementes D-Pulvers.

Der erfindungsgemäße PHA kann nicht nur zur Herstellung von Biozement D, sondern auch zur Herstellung der Zementmischungen F und H verwendet werden. Die Zusammensetzungen und Mischungsverhältnisse der Biozemente D, F und H sind aus der WO 99/49906 bekannt. Wie vorher schon erwähnt, wurde in diesen Biozementen jedoch ein PHA anderer Zusammensetzung verwendet.

In einer bevorzugten Ausführungsform beträgt der Anteil des PHA 1 bis 5 Gew. % bezogen auf die Gesamttrockenmasse. Noch bevorzugter beträgt der PHA-Anteil 1.7 bis 2.7 Gew.% bezogen auf die Gesamttrockenmasse des Biozementes.

Geeignete Verbindungen, die zu TCP hinzugemischt werden können, sind generell alle anorganischen Verbindungen, die Calcium und Phosphat enthalten. Bevorzugt werden die Verbindungen, die aus der folgenden Gruppe ausgewählt werden:

$CaHPO_4$, carbonathaltiger Apatit und $CaCO_3$.

Die erfindungsgemäßen Mischungen können, falls gewünscht, auch bekannte Abbindungsbeschleuniger enthalten. Bevorzugt ist hierbei Dinatriumhydrogenphosphat.

Ferner ist es wünschenswert der Mischung pharmazeutische Wirkstoffe beizumischen, welche die unterschiedlichsten Wirkungen besitzen. Beispiele solcher Wirkstoffe sind Wachstumsfaktoren wie FGF (Fibroblast Growth Factor) , BMP (Bone Morphogenetic Protein) oder TGF-ß (Tissue Growth Factor) oder andere Wirkstoffe wie Prostaglandine. Die Biozemente sind aufgrund ihrer Struktur in der Lage, die Wirkstoffe innerhalb von einigen Tagen nach der Implantierung in die Umgebung abzugeben.

Weiterhin ist es sinnvoll der erfindungsgemäßen Mischung als temporärer Schutz vor Keimbesiedlung während der Implantation Antibiotika oder Desinfektionsmittel zuzusetzen, analog zu den bekannten Mischungen nach WO 99/49906.

Gegenstand der Erfindung ist auch eine entsprechende Mischung in Form einer wässrigen Lösung, Paste oder Suspension sowie ihre Verwendung zur Herstellung von biologisch abbaubaren implantierbaren synthetischen Knochenmaterialien.

Der PHA wird nach folgendem Beispiel hergestellt.

Beispiel:

Es werden drei Salze mit folgenden Mengenangaben zusammengegeben und homogen vermischt.

$$40.67 \text{ g } CaSO_4 \cdot 2H_2O + 60.0 \text{ g } Na_3PO_4 \cdot 12H_2O + 0.96 \text{ g } CaCO_3$$

Diese Mischung wird in ein 600 ml Becherglas überführt. Anschließend wird 200 ml einer wässrigen Lösung bestehend aus 20 g $Na_2HPO_4 \cdot 2H_2O$ + 5 g $MgCl_2 \cdot 6H_2O$ + 20 g $K_2HPO_4$ pro 1000 ml zugeführt. Die Lösung wird bei Raumtemperatur 2 h gerührt. Das Präzipitat wird mittels Filtration von der Lösung getrennt. Danach wird das Präzipitat 20 mal mit jeweils 50 ml einer 0.9%igen NaCl Lösung gewaschen. Anschließend erfolgt die Trocknung des Präzipitats über Nacht bei 120° C. Eine Aggregation wird nicht beobachtet. Das Röntgendiffraktogramm ergab die Struktur eines Mikroapatits. Das FT-IR Spektrum zeigte charakteristische Apatit- und Carbonat-Bindungen vom B-Typ.

Tab.1 zeigt die Kompressionsfestigkeiten (in MPa) der vorliegenden Erfindung nach 2, 4, 6, 18, 72 und 240 Stunden im Vergleich zur WO 99/49906.

Tab.1

| Zeit [h] | Kompressionsfestigkeit WO 99/49906 | Kompressionsfestigkeit Erfindung |
|----------|-----------------------------------|----------------------------------|
| 2 | 16 | |
| 4 | 26 | |
| 6 | | 29.2 |
| 18 | 45 | 46 |
| 72 | 47 | 74.3 |
| 240 | 48 | 75.5 |

In Figur 1 sind die Werte aus Tabelle 1 grafisch dargestellt.

Die Ergebnisse zeigen, dass die der Erfindung zugrundeliegende Aufgabenstellung gelöst ist und die Kompressionsfestigkeit des erfindungsgemäßen PHA nach etwa 48 h schon deutlich höhere Werte gegenüber dem Stand der Technik zeigt.

Die Bestimmung der Kompressionsfestigkeit erfolgte mit einer Materialprüfmaschine Lloyd Typ LR50K nach 2, 4, 6, 18, 72 und 240 Stunden Immersion in Ringer-Lösung. Das Reaktionsprodukt wird mittels Röntgendiffraktometrie bestimmt.

Fig.1

4

**Patentansprüche**

1. Mischung von Pulvern geeignet zur Herstellung von resorbierbaren Calciumphophat-Zementen, enthaltend Tricalciumphosphat (TCP), wobei 30- 70% der TCP-Teilchen eine Partikelgröße von 0.1 bis 7 µm und 10 - 60% eine Partikelgröße von 40 - 100 µm aufweisen, präzipitierter Hydroxylapatit (PHA) und mindestens einer weiteren anderen phosphathaltigen anorganischen Verbindung, **dadurch gekennzeichnet, dass** der PHA ein Kation-defizienter Hydroxylapatit der Formel I

$$Ca_{8.75}V(Ca)_{1.25}[(HPO_4)_{5.5-x}(CO_3)_{0.5}](OH)_x V(OH)_{2-x} \text{ mit } x = 0\text{-}2 \text{ ist.}$$

2. Mischung nach Anspruch 1, **dadurch gekennzeichnet, dass** sie nach der Aushärtung eine Kompressionsfestigkeit zwischen 70 und 80 MPa besitzt.

3. Mischung nach Anspruch 1 und/oder 2, **dadurch gekennzeichnet, dass** der PHA eine Partikelgröße von 0.5 bis 10 µm besitzt.

4. Mischung nach einem der Ansprüche 1 bis 3, **dadurch gekennzeichnet, dass** der PHA eine Partikelgröße von 0.5 bis 5 µm besitzt.

5. Mischung nach einem der Ansprüche 1 bis 4, **dadurch gekennzeichnet, dass** der Anteil des PHA 1 bis 5 Gew. % bezogen auf die Gesamttrockenmasse beträgt.

6. Mischung nach einem der Ansprüche 1 bis 5, **dadurch gekennzeichnet, dass** der Anteil des PHA 1.7 bis 2.7 Gew.% bezogen auf die Gesamttrockenmasse beträgt.

7. Mischung nach einem der Ansprüche 1 bis 6, **dadurch gekennzeichnet, dass** mindestens eine andere phosphathaltige anorganische Verbindung ausgewählt wurde aus der Gruppe:
   $CaHPO_4$, carbonathaltiger Apatit und $CaCO_3$.

8. Mischung nach einen der Ansprüche 1 bis 7, **dadurch gekennzeichnet, dass** sie zusätzlich einen Abbindungsbeschleuniger enthält.

9. Mischung nach einen der Ansprüche 1 bis 8, **dadurch gekennzeichnet, dass** sie zusätzlich einen pharmazeutischen Wirkstoff enthält.

10. Mischung nach Anspruch 9, **dadurch gekennzeichnet, dass** sie zusätzlich ein Antibiotikum oder ein Desinfektionsmittel enthält.

11. Mischung nach einen der Ansprüche 1 bis 10, **dadurch gekennzeichnet, dass** sie in Form einer wässrigen Lösung, Suspension oder Paste vorliegt.

12. Biologisch abbaubares Implantat, hergestellt aus einer ausgehärteten Mischung nach Anspruch 11.

13. Verwendung einer Mischung nach Anspruch 11 zur Herstellung von biologisch abbaubaren, implantierbaren, synthetischen Knochenmaterialien.

**Europäisches Patentamt**

## EUROPÄISCHER RECHERCHENBERICHT

Nummer der Anmeldung

EP 00 11 0045

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y,D | DE 198 13 614 A (MERCK PATENT GMBH) 30. September 1999 (1999-09-30) * das ganze Dokument * | 1-13 | A61L27/12 A61L24/02 A61K6/033 |
| Y | EP 0 422 719 A (LEDARD CLAUDE) 17. April 1991 (1991-04-17) * Zusammenfassung * * Spalte 1 - Spalte 2 * | 1-13 | |
| A | DE 197 41 286 A (DEGUSSA) 2. April 1998 (1998-04-02) * Zusammenfassung * * Seite 2, Zeile 11 * * Seite 3, Zeile 24-63 * * Seite 5, Zeile 51-57 * * Seite 6, Zeile 10 - Seite 7, Zeile 50 * | 1,3-7, 11-13 | |
| A | EP 0 950 420 A (MERCK PATENT GMBH) 20. Oktober 1999 (1999-10-20) * Seite 2, Zeile 10-19 * * Seite 3, Zeile 27 - Seite 4, Zeile 6 * * Beispiele 1,6 * | 1,2,5-8, 11,13 | |

**RECHERCHIERTE SACHGEBIETE (Int.Cl.7)**

A61L
A61K

Der vorliegende Recherchenbericht wurde für alle Patentansprüche erstellt

| Recherchenort | Abschlußdatum der Recherche | Prüfer |
|---|---|---|
| DEN HAAG | 20. Oktober 2000 | Böhm, I |

KATEGORIE DER GENANNTEN DOKUMENTE

X : von besonderer Bedeutung allein betrachtet
Y : von besonderer Bedeutung in Verbindung mit einer
     anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
     nach dem Anmeldedatum veröffentlicht worden ist
D : in der Anmeldung angeführtes Dokument
L : aus anderen Gründen angeführtes Dokument

............................................................
& : Mitglied der gleichen Patentfamilie, übereinstimmendes
     Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 00 11 0045

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-10-2000

| Im Recherchenbericht angeführtes Patentdokument | | | Datum der Veröffentlichung | Mitglied(er) der Patentfamilie | | | Datum der Veröffentlichung |
|---|---|---|---|---|---|---|---|
| DE 19813614 | A | | 30-09-1999 | AU | 2933699 | A | 18-10-1999 |
| | | | | WO | 9949906 | A | 07-10-1999 |
| EP 0422719 | A | | 17-04-1991 | FR | 2652748 | A | 12-04-1991 |
| | | | | DE | 69005074 | D | 20-01-1994 |
| | | | | DE | 69005074 | T | 23-06-1994 |
| | | | | ES | 2062308 | T | 16-12-1994 |
| | | | | US | 5141561 | A | 25-08-1992 |
| DE 19741286 | A | | 02-04-1998 | AU | 719417 | B | 11-05-2000 |
| | | | | AU | 3931897 | A | 02-04-1998 |
| | | | | BR | 9704938 | A | 25-05-1999 |
| | | | | EP | 0832636 | A | 01-04-1998 |
| | | | | JP | 10114617 | A | 06-05-1998 |
| | | | | US | 5952399 | A | 14-09-1999 |
| EP 0950420 | A | | 20-10-1999 | DE | 19816858 | A | 21-10-1999 |
| | | | | JP | 2000000295 | A | 07-01-2000 |

EPO FORM P0461

Für nähere Einzelheiten zu diesem Anhang : siehe Amtsblatt des Europäischen Patentamts, Nr.12/82